(19) 

Europäisches
Patentamt
European
Patent Office
Office européen
des brevets

(11) **EP 4 279 055 B1**

(12) **EUROPEAN PATENT SPECIFICATION**

(45) Date of publication and mention
of the grant of the patent:
**20.08.2025 Bulletin 2025/34**

(21) Application number: **22739489.7**

(22) Date of filing: **14.01.2022**

(51) International Patent Classification (IPC):
*A61K 8/02* *(2006.01)*     *A61K 8/19* *(2006.01)*
*A61K 8/31* *(2006.01)*     *A61K 8/86* *(2006.01)*
*A61Q 1/12* *(2006.01)*

(52) Cooperative Patent Classification (CPC):
**A61Q 1/12; A61K 8/8111; A61K 8/86**

(86) International application number:
**PCT/JP2022/001134**

(87) International publication number:
**WO 2022/154084 (21.07.2022 Gazette 2022/29)**

(54) **METHOD FOR PRODUCING SOLID POWDER COSMETIC**

VERFAHREN ZUR HERSTELLUNG EINES KOSMETIKUMS MIT FESTEM PULVER

PROCÉDÉ DE PRODUCTION D'UN PRODUIT COSMÉTIQUE EN POUDRE SOLIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **14.01.2021   JP 2021004557**

(43) Date of publication of application:
**22.11.2023   Bulletin 2023/47**

(73) Proprietor: **Kao Corporation**
**Chuo-ku**
**Tokyo 103-8210 (JP)**

(72) Inventors:
 • **OTA, Ko**
   **Odawara-shi, Kanagawa 250-0002 (JP)**

 • **YAMAWAKI, Kenji**
   **Odawara-shi, Kanagawa 250-0002 (JP)**
 • **ABE, Tomoaki**
   **Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

(56) References cited:
**JP-A- 2009 242 282     JP-A- 2009 242 282
JP-A- 2009 242 283     JP-A- 2010 047 528
JP-A- 2016 185 912     JP-A- 2020 183 352
JP-A- H08 208 432     US-A- 5 958 389
US-A1- 2005 069 565     US-A1- 2018 028 414**

**EP 4 279 055 B1**

**Description**

Field of the Invention

The present invention relates to a method for producing a solid powder cosmetic.

Background of the Invention

Methods for producing solid powder cosmetics are divided into dry methods including compression molding an oil powder bulk obtained by mixing a powder and an oil agent, and wet methods including adding water or a volatile solvent such as an alcohol to an oil powder bulk to make a slurry, packing the slurry in a container, and thereafter, removing the volatile solvent through drying to thereby obtain cosmetics. The cosmetics obtained by the wet methods are characterized by having the fine powder texture and the moist use impression due to the improved homogeneous dispersion of the powder and oil agent, as compared with those obtained by the dry methods.

Volatile hydrocarbons and low-boiling point alcohols have been used as the solvents for dispersion in the wet methods; however, in recent years, from the growing environmental consciousness, methods involving use of water as a solvent have been reported. In the case of preparing a slurry by using water, however, homogeneous dispersion of the oil agent is difficult, and accordingly, the oil agent is dispersed in an inhomogeneous state from the surface to the interior of a product obtained, posing problems in the usability and the homogeneity of molded products.

As a method of improving the dispersibility of an oil agent even when water is used as a solvent for dispersion, there is reported a method including previously making an oil component into a form of an oil-in-water emulsion composition; mixing the composition with a powder to make a slurry; packing the slurry in a container, and thereafter removing water through drying to thereby obtain a solid powder cosmetic (Patent Literature 1).

Furthermore, US 5,958,389 relates to a cosmetic composition in the form of a compact powder comprising a fatty phase and a pulverulent phase. Said pulverulent phase comprises a first filler and at least a second filler. The composition is obtained by preparing an oil-in-water type emulsion of the fatty phase in an aqueous phase, dispersing the pulverulent phase in the emulsion, casting the dispersion obtained in a mould, and freeze drying the dispersion.

US 2018/028414 A1, JP 2020-183352 A, and JP 2010-047528 A inter alia describe methods for producing solid powder cosmetics, wherein a pigment composition is mixed with oil agents followed by the addition of water.

Patent Literature 1: JP-A-2009-242282

Summary of the Invention

The present invention is a method for producing a solid powder cosmetic comprising the following components (A) to (C):

> (A) a color pigment in an amount of 0.1 mass% or higher and 34 mass% or lower in the whole of powder;
> (B) an oil agent in an amount of 3 mass% or higher and 28 mass% or lower with respect to the whole of powder; and
> (C) a surfactant in an amount of 12 mass% or higher and 30 mass% or lower with respect to the total amount of the oil agent,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

Detailed Description of the Invention

The problems of the solid powder cosmetic obtained by the method of Patent Literature 1 are that roughness is liable to be caused on the surface of a molded product due to drying; the amount of the solid powder cosmetic taken upon application is nonconstant; the surface of molded product is hard; and the powder aggregates, easily giving much powder texture upon use.

Therefore, the present invention relates to a method for producing a solid powder cosmetic which is high in the surface uniformity of molded articles, appropriate in terms of the amount taken upon application, very smooth and comfortable and gives no feeling of powder, even in the case where water has been used as a solvent for dispersion.

As a result of studies on the step of packing a cosmetic to solve the above problems, the present inventors found that the point lies in a composition of an oil-in-water emulsion prepared by using water as a solvent for dispersion before mixing with a powder, and also in the step of removing the solvent. Specifically, the present inventors found that a solid powder cosmetic which is high in the surface uniformity of molded products, appropriate in terms of the amount taken upon application, very smooth and comfortable and gives no feeling of powder can be obtained by mixing powdery cosmetic bases with an oil-in-water emulsion prepared by using a specific amount of a surfactant to make a slurry; packing the slurry in a cosmetic container; thereafter, removing the solvent by suction; and completed the present invention.

According to the present invention, there can be produced the solid powder cosmetic which is high in the surface uniformity of molded products, appropriate in terms of the amount taken upon application, very smooth and comfortable and gives no feeling of powder.

The solid powder cosmetic according to the production method of the present invention contains the following components (A) to (C):

(A) a color pigment in an amount of 0.1 mass% or higher and 34 mass% or lower in the whole of powder;
(B) an oil agent in an amount of 3 mass% or higher and 28 mass% or lower with respect to the whole of powder; and
(C) a surfactant in an amount of 12 mass% or higher and 30 mass% or lower with respect to the total amount of the oil agent.

Herein, the content of a component means the mass proportion of the component to the substance(s) as a basis, the mass of which is regarded as 100, and is expressed in "mass%" as unit.

The solid powder cosmetic produced according to the present invention contains a powder containing the color pigment (A).

The powder to be used in the present invention is not limited as long as it is one to be used for usual cosmetics, and may include an extender pigment, a glittering pigment and the like, in addition to the color pigment.

Examples of the color pigment (A) include metal oxides such as titanium oxide, zinc oxide, yellow iron oxide, red iron oxide, black iron oxide, Berlin blue, ultramarine blue, chrome oxide and chromium hydroxide; metal complexes such as manganese violet and cobalt titanate; inorganic pigments such as carbon black; synthetic organic pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1 and Blue No. 404; and natural organic coloring matter such as β-carotene, caramel and paprika coloring matter.

Examples of the extender pigment include inorganic pigments such as silicic acid, silicic acid anhydride, magnesium silicate, talc, sericite, boron nitride, mica, synthetic mica, glass flake, synthetic phlogopite, kaolin, clay, bentonite, bismuth oxychloride, zirconium oxide, magnesium oxide, aluminum oxide, calcium sulfate, barium sulfate, magnesium sulfate, calcium carbonate, magnesium carbonate, aluminum silicate, magnesium silicate, silica and alumina, and composite powders of these.

As the glittering pigment, there can be used a platy powder or the like of mica, synthetic phlogopite, glass, silica, alumina or the like coated with a coloring agent such as titanium oxide, iron oxide, silicon oxide, Berlin blue, chromium oxide, tin oxide, chromium hydroxide, gold, silver, carmine or an organic pigment, on their surface; a powder of a raw film cut into an optional shape, such as a polyethylene terephthalate·polymethyl methacrylate laminated powder, a polyethylene terephthalate·aluminum-deposited powder or a polyethylene terephthalate·gold-deposited powder; or the like.

Further, there can be used an organic powder such as a polyethylene powder, polypropylene powder, polymethyl methacrylate powder, nylon powder, polytetrafluoroethylene powder, silicone powder, silicone rubber powder, silk powder, urethane powder, cellulose powder, starch powder or polyfluoroethylene powder; an inorganic powder such as silica, magnesium carbonate, or calcium carbonate; acylated lysine powder such as lauroyl lysine; a metallic soap powder being a higher fatty acid metal salt; or the like.

Then, the above-mentioned organic powders and inorganic powders are preferably globular powder, in view of appropriately taking the powder of the solid powder cosmetic and the favorably spreading to skin. More preferred is combination use of the globular powder with at least any one pigment among the extender pigment, the color pigment and the glittering pigment.

These powders are not limited in terms of the size, the shape and the like. They can be used as they are, or can be subjected to hydrophobization treatment or hydrophilization treatment by usual methods, before use.

The hydrophobization treatment is not limited as long as it is a treatment usually applied to powders for cosmetics, and may be carried out as a dry treatment, a wet treatment or the like by using a surface treating agent such as a fluoro compound, silicone-based compound, metallic soap, amino acid-based compound, lecithin, alkyl silane, oil agent, or organic titanate.

Specific examples of the surface treating agent include fluoro compounds such as perfluoropolyether, perfluoroalkyl phosphates, perfluoroalkylalkoxysilane and fluorine-modified silicones; silicone-based compounds such as dimethylpolysiloxane, methylhydrogenpolysiloxane, cyclic silicones, one-end or both-end trialkoxy group-modified organopolysiloxane, crosslinked silicone, silicone resins, fluorine-modified silicone resins and acryl-modified silicone; metallic soaps such as aluminum stearate, aluminum myristate, zinc stearate and magnesium stearate; amino acid-based compounds such as proline, hydroxyproline, alanine, glycine, sarcosine, glutamic acid, aspartic acid, lauroyllysine, lysine and derivatives of these, and acylated amino acids; lecithin and hydrogenated lecithin; alkylsilanes such as methyltrimethoxysilane, ethyltrimethoxysilane, hexyltrimethoxysilane, octyltrimethoxysilane, octyltriethoxysilane and triethoxycaprylylsilane; oil agents such as polyisobutylene, waxes, fats and oils and fatty acids; and organic titanates such as isopropyl triisostearoyl titanate.

Then, the hydrophilization treatment is not limited as long as it is a treatment usually applied to powders for cosmetics.

Examples therefor include plant-derived polymers such as gum arabic, gum tragacanth, arabinogalactan, locust bean gum (carob gum), guar gum, karaya gum, carrageenan, pectin, agar, quince seed (marmelo), starch (rice, corn, potato, wheat), algae colloid, tranto gum and locust bean gum; microbial polymers such as xanthan gum, dextran, succinoglycan and pullulan; animal-derived polymers such as collagen, casein, albumin, deoxyribonucleic acids (DNA), and salts thereof; starch-based polymers such as carboxymethyl starch and methylhydroxypropyl starch; cellulose-based polymers such as methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, crystalline cellulose and cellulose powders; alginic acid-based polymers such as sodium alginate and propylene glycol alginate; vinyl-based polymers such as polyvinyl methyl ether, polyvinylpyrrolidone, carboxyvinyl polymers; polyoxyethylene-based polymers such as polyethylene glycol and polyethylene glycol silane; polyoxyethylene polyoxypropylene copolymer-based polymers; acrylic polymers such as sodium polyacrylate, polyethyl acrylate and polyacrylic acid amide; and further, inorganic silicate-based compounds such as silica.

In view of taking the powder, the spreadability while applying, and the makeup persistency, the powder is preferably one containing a hydrophobic powder, and more preferably one surface-treated with a fluoro compound, silicone-based compound, metallic soap, amino acid-based compound, lecithin, alkylsilane, oil agent, organic titanate or the like.

In the case of containing the above-mentioned hydrophobic powder as the powder, the content of the hydrophobic powder in the whole powder is preferably 10 mass% or higher, more preferably 30 mass% or higher and further more preferably 50 mass% or higher, and preferably 100 mass% or lower, more preferably 95 mass% or lower and further more preferably 90 mass% or lower, since the hydrophobic powder is very smooth and comfortable and gives no feeling of powder.

The powders can be used singly or in a combination of two or more thereof; and the total content of the powder(s) in the solid powder cosmetic produced according to the present invention is preferably 70 mass% or higher and 95 mass% or lower, more preferably 72 mass% or higher and 92 mass% or lower, further more preferably 74 mass% or higher and 90 mass% or lower and even more preferably 75 mass% or higher and 85 mass% or lower, in the total amount of the solid powder cosmetic in view of the spreadability upon applying, the moist feeling upon applying, and the makeup persistency.

The color pigment (A) is contained in an amount of **0.1** mass% or higher and 34 mass% or lower in the whole of powder, in view of providing the solid powder cosmetic which is appropriate in terms of the amount taken upon application, very smooth and comfortable and gives no feeling of powder.

The amount is preferably **0.2** mass% or higher, more preferably **0.3** mass% or higher, even more preferably **0.5** mass% or higher, even more preferably **1.0** mass% or higher and even more preferably 2 mass% or higher, and preferably 30 mass% or lower and more preferably 25 mass% or lower.

It is preferable that the extender pigment be contained in an amount of 5 mass% or higher and 95 mass% or lower in the whole of powder, in view of providing the solid powder cosmetic which is high in the moldability and the surface uniformity of molded products, appropriate in terms of the amount taken upon application, very smooth and comfortable and gives no feeling of powder.

In view of providing the solid powder cosmetic which is high in the moldability, very smooth and comfortable and gives no feeling of powder, the amount is more preferably 10 mass% or higher, further more preferably 15 mass% or higher and even more preferably 20 mass% or higher; and in view of enhancing the surface uniformity of molded products, the amount is more preferably 85 mass% or lower, further more preferably 75 mass% or lower and even more preferably 65 mass% or lower.

It is preferable that the glittering pigment be contained in an amount of 0 mass% or higher and 95 mass% or lower in the whole of powder, in view of providing the solid powder cosmetic which is high in the surface uniformity of molded products, appropriate in terms of the amount taken upon application, very smooth and comfortable and gives no feeling of powder. In view of enhancing the surface uniformity of molded articles, the amount is more preferably 5 mass% or higher, further more preferably 10 mass% or higher and even more preferably 20 mass% or higher; and in view of the moldability, the amount is more preferably 85 mass% or lower, further more preferably 75 mass% or lower and even more preferably 65 mass% or lower.

The solid powder cosmetic produced according to the present invention contains the oil agent (B).

Examples of the oil agent include hydrocarbon oils, ester oils, ether oils, silicone oils and higher alcohols having 10 to 24 carbon atoms.

The hydrocarbon oils specifically include squalane, liquid paraffin, liquid isoparaffin and heavy liquid isoparaffin.

Among these hydrocarbon oils, it is preferable to contain at least one or more selected from the group consisting of liquid paraffin, liquid isoparaffin and squalene, and it is more preferable to contain at least one selected from the group consisting of liquid isoparaffin and squalene, in view of providing the solid powder cosmetic which is high in the surface uniformity of molded products, appropriate in terms of the amount taken upon application, very smooth and comfortable and gives no feeling of powder.

The ester oils include monoester oils, diester oils, trieste oils and tetraester oils.

The monoester oils include monoesters of aliphatic or aromatic monocarboxylic acids or dicarboxylic acids having 2 to 24 carbon atoms; and specific examples thereof include cetyl 2-ethylhexanoate, cetyl octanoate, isononyl isononanoate, isotridecyl isononanoate, hexyl laurate, isopropyl myristate, octyldodecyl myristate, myristyl myristate, 2-hexyldecyl myristate, isopropyl palmitate, octyl palmitate, 2-hexyldecyl palmitate, butyl stearate, isocetyl stearate, isocetyl isostearate, decyl oleate, isodecyl benzoate, octyl methoxycinnamate, hexyldecyl dimethyloctanoate, cetyl lactate, myristyl lactate, lanolin acetate, 2-ethylhexyl succinate, 2-hexyldecyl adipate and alkyl (C12-C15) benzoates. Among these, it is preferable to contain at least one or more selected from the group consisting of cetyl ethylhexanoate, isononyl isononanoate, isotridecyl isononanoate, isopropyl myristate and octyl methoxycinnamate; it is more preferable to contain at least one or more selected from the group consisting of cetyl ethylhexanoate, isononyl isononanoate and isotridecyl isononanoate; and it is further more preferable to contain at least one or more selected from the group consisting of cetyl ethylhexanoate and isononyl isononanoate, in view of providing the solid powder cosmetic which is high in the surface uniformity of molded products, appropriate in terms of the amount taken upon application, very smooth and comfortable and gives no feeling of powder.

The diester oils include diesters of dicarboxylic acids having 3 to 18 carbon atoms, and di-fatty acid esters of polyhydric alcohols. Specific examples thereof include propylene glycol dicaprylate, neopentyl glycol dicaprate, glycol distearate, propylene glycol diisostearate, glyceryl diisostearate, glyceryl monoisostearate monomyristate, glycerol di-2-heptylundecanoate, di-2-ethylhexyl succinate, diisopropyl sebacate, diisostearyl malate, ethylene glycol 2-ethylhexanoate, diisobutyl adipate, di-2-heptylundecyl adipate and di-2-ethylhexyl sebacate. Among these, it is preferable to contain at least one or more selected from the group consisting of propylene glycol dicaprylate, neopentyl glycol dicaprate, glycol distearate, diisostearyl malate and propylene glycol diisostearate; it is more preferable to contain at least one or more selected from the group consisting of propylene glycol dicaprylate, neopentyl glycol dicaprate and diisostearyl malate; and it is further more preferable to contain at least one or more selected from the group consisting of neopentyl glycol dicaprate and diisostearyl malate; and it is even more preferable to contain diisostearyl malate, in view of providing the solid powder cosmetic which is high in the surface uniformity of molded products, appropriate in terms of the amount taken upon application, very smooth and comfortable and gives no feeling of powder.

The triester oils include tri-fatty acid esters of tri- or higher polyhydric alcohols. Specific examples thereof include glycerol trimyristate, glycerol triisopalmitate, glycerol tri-2-heptylundecanoate, trimethylolpropane triethylhexanoate, trimethylolpropane trioctanoate, glycerol tri-(caprylate·caprate), glycerol trioleate, glycerol tri-2-ethylhexanoate, glycerol triisostearate, olive oil and jojoba oil. Among these, it is preferable to contain at least one or more selected from the group consisting of glycerol tri-(caprylate·caprate), glycerol trioleate, glycerol tri-2-ethylhexanoate and glycerol triisostearate; and it is more preferable to contain glycerol tri-(caprylate·caprate), in view of providing the solid powder cosmetic which is high in the surface uniformity of molded products, appropriate in terms of the amount taken upon application, very smooth and comfortable and gives no feeling of powder.

The tetraester oils include tetra-fatty acid esters of tetra- or higher polyhydric alcohols. Specific examples thereof include pentaerythritol tetra-(behenate/ benzoate/ethylhexanoate), pentaerythritol tetraethylhexanoate, pentaerythritol tetraoctanoate and pentaerythritol tetra-2-ethylhexanoate. Among these, it is preferable to contain at least one or more selected from the group consisting of pentaerythritol tetraethylhexanoate, pentaerythritol tetraoctanoate and pentaerythritol tetra-2-ethylhexanoate; and it is more preferable to contain pentaerythritol tetraethylhexanoate, in view of providing the solid powder cosmetic which is high in the surface uniformity of molded products, appropriate in terms of the amount taken upon application, very smooth and comfortable and gives no feeling of powder.

The ether oils include dialkyl ethers, and specifically include dihexyl ether, dicaprylyl ether and cetyl-1,3-dimethyl butyl ether. Among these, it is preferable to contain at least one or more selected from the group consisting of dicaprylyl ether and cetyl-1,3-dimethyl butyl ether; and it is more preferable to contain cetyl-1,3-dimethyl butyl ether, in view of providing the solid powder cosmetic which is high in the surface uniformity of molded products, appropriate in terms of the amount taken upon application, very smooth and comfortable and gives no feeling of powder.

The silicone oils include crosslinked methylpolysiloxane, network methylpolysiloxane, dimethylpolysiloxane, methyl trimethicone, dimethylcyclopolysiloxane and methylphenylpolysiloxane such as diphenylsiloxyphenyl trimethicone and higher alcohol-modified organopolysiloxanes. Among these, it is preferable to contain at least one or more selected from the group consisting of dimethylpolysiloxane, methyl trimethicone, dimethylcyclopolysiloxane and methylphenylpolysiloxane; it is more preferable to contain at least one or more selected from the group consisting of dimethylpolysiloxane, methyl trimethicone and methylphenylpolysiloxane; and it is further more preferable to contain at least one or more selected from the group consisting of dimethylpolysiloxane, methyl trimethicone and diphenylsiloxyphenyl trimethicone, in view of providing the solid powder cosmetic which is high in the surface uniformity of molded products, appropriate in terms of the amount taken upon application, very smooth and comfortable and gives no feeling of powder. The silicone oil even more preferably contains a volatile silicone oil.

Examples of the volatile silicone oil include linear dimethylpolysiloxanes such as dimethylpolysiloxane (1 cs), dimethylpolysiloxane (1.5 cs) and dimethylpolysiloxane (2 cs); branched siloxanes such as methyl trimethicone, tris(trimethylsilyl)methylsilane and tetrakis(trimethylsilyl)silane; and cyclic dimethylsiloxanes such as octamethylcyclotetrasiloxane, decamethylcyclopentasiloxane and dodecamethylcyclohexasiloxane; it is preferable to contain at least one or more selected from the group consisting of linear dimethylpolysiloxanes and branched siloxanes; and it is more preferable to contain at least one or more selected from the group consisting of dimethylpolysiloxane (1.5 cs) and methyl trimethicone.

For the higher alcohols having 10 to 24 carbon atoms, it is preferable to contain at least one or more selected from the group consisting of higher alcohols having 12 to 22 carbon atoms; it is more preferable to contain at least one or more selected from the group consisting of higher alcohols having 16 to 22 carbon atoms; it is further more preferable to contain at least one or more selected from the group consisting of higher alcohols having 18 to 22 carbon atoms; and it is even more preferable to contain at least one or more selected from the group consisting of higher alcohols having 18 or 22 carbon atoms, in view of the stability of the oil-in-water emulsion, the stability of the slurry, and the like.

For the oil agent of the component (B), it is preferable to contain at least one or more selected from the group consisting of the ester oils and the silicone oils; it is more preferable to contain at least one or more selected from the group consisting of the monoester oils, the diester oils and the silicone oils; and it is further preferable to contain at least one or more selected from the group consisting of the monoester oils, the diester oils, dimethylpolysiloxane, methyl trimethicone and methylphenylpolysiloxane, in view of providing the solid powder cosmetic which is high in the surface uniformity of molded products, appropriate in terms of the amount taken upon application, very smooth and comfortable and gives no feeling of powder.

In view of providing the solid powder cosmetic which is high in the surface uniformity of molded products, appropriate in terms of the amount taken upon application, very smooth and comfortable and gives no feeling of powder, the oil agent of the component (B) is preferably a liquid at 25°C. The oil agent of the component (B) more preferably contains at least the volatile silicone oil.

The content of the component (B) is 3 mass% or higher and 28 mass% or lower with respect to the whole of powder, and in view of providing the solid powder cosmetic which is high in the surface uniformity of molded products, appropriate in terms of the amount taken upon application, very smooth and comfortable and gives no feeling of powder, the content is preferably 5 mass% or higher and 27 mass% or lower. Further in view of providing the solid powder cosmetic which is appropriate in the moldability and in the amount taken upon application, very smooth and comfortable and gives no feeling of powder, the content is more preferably 7 mass% or higher, further more preferably 10 mass% or higher, even more preferably 14 mass% or higher and even more preferably 18 mass% or higher. Further in view of providing the solid powder cosmetic which is high in the surface uniformity of molded products, appropriate in terms of the amount taken upon application, very smooth and comfortable and gives no feeling of powder, the content is 28 mass% or lower.

In view of the stability of the slurry, the surfactant of the component (C) preferably contains a nonionic surfactant.

Examples of the surfactant of the component (C) includes nonionic surfactants other than silicone-based ones, and silicone-based nonionic surfactants.

The nonionic surfactants other than silicone-based ones are not limited as long as they are ones used for usual cosmetics, and examples thereof include sorbitan fatty acid esters such as sorbitan monoisostearate, glycerol fatty acid esters, polyglycerol fatty acid esters, propylene glycol fatty acid esters, polyethylene glycol fatty acid esters, sucrose fatty acid esters, polyoxyethylene fatty acid esters, polyoxyethylene sorbitan fatty acid esters, polyoxyethylene alkyl ethers, polyoxyethylene sorbitol fatty acid esters, polyoxyethylene glycerol fatty acid esters, polyoxyethylene propylene glycol fatty acid esters, polyoxyethylene castor oils, polyoxyethylene hydrogenated castor oils, polyoxyethylene hydrogenated castor oil fatty acid esters, polyoxyethylene phytostanol ethers, polyoxyethylene phytosterol ethers, polyoxyethylene cholestanol ethers and polyoxyethylene cholesteryl ethers.

The silicone-based nonionic surfactants include linear or branched polyether-modified silicones such as polyoxyethylene·methylpolysiloxane copolymers and polyoxyethylene·polydimethylsiloxyethyl dimethicone (specifically, PEG-9 polydimethylsiloxyethyl dimethicone); alkyl-modified polyether-modified silicones (specifically, cetyl PEG/PPG-10/1 dimethicone); and alkyl chain-comodified polyether-modified silicones such as alkyl-modified polyoxyethylene·polydimethylsiloxyethyl dimethicone (specifically, lauryl PEG-9 polydimethylsiloxyethyl dimethicone).

Among these, it is preferable to contain at least one or more selected from the group consisting of sorbitan fatty acid esters, linear or branched polyether-modified silicones, alkyl-modified polyether-modified silicones and alkyl-modified polyoxyethylene·polydimethylsiloxyethyl dimethicone; it is more preferable to contain at least one or more selected from the group consisting of sorbitan fatty acid esters, linear or branched polyether-modified silicones and alkyl-modified polyether-modified silicones; and it is further more preferable to contain at least one or more selected from the group consisting of sorbitan fatty acid esters and alkyl-modified polyether-modified silicones, in view of the stability of the oil-in-water emulsion, the stability of the slurry, and the provision of the solid powder cosmetic which is high in the surface uniformity of molded products, appropriate in terms of the amount taken upon application, very smooth and comfortable and gives no feeling of powder. Further it is preferable to use the nonionic surfactant other than a silicone-based one together with the silicone-based nonionic surfactant in combination.

For these nonionic surfactants, the HLB is preferably from 3.5 to 16, more preferably from 7 to 14 and further more preferably from 8 to 12, in view of the stability of the oil-in-water emulsion, the stability of the slurry, and the provision of the solid powder cosmetic which is high in the surface uniformity of molded products, appropriate in terms of the amount taken upon application, very smooth and comfortable and gives no feeling of powder.

Here, the HLB (Hydrophilic-Lipophilic Balance) indicates a molecular weight of the hydrophilic group moiety in the entire molecular weight of a surfactant, and is determined by Griffin equation in the case of a nonionic surfactant. The HLB of a mixed surfactant constituted of two or more of nonionic surfactants is determined as follows. The HLB of the mixed surfactant is a value obtained by arithmetically averaging HLB values of each nonionic surfactant based on their blend ratio.

$$\mathrm{Mixed\ HLB} = \Sigma(\mathrm{HLBx} \times \mathrm{Wx})/\Sigma \mathrm{Wx}$$

wherein HLBx represents an HLB value of a nonionic surfactant X; and Wx represents a weight (g) of the nonionic surfactant X having the value of HLBx.

In the case of using a nonionic surfactant other than silicone-based ones together with a silicone-based nonionic surfactant in combination as the component (C), the mass ratio of the silicone-based nonionic surfactant to the nonionic surfactant other than silicone-based ones is preferably (silicone-based nonionic surfactant)/(nonionic surfactant other than silicone-based ones) = from 0.5 to 20, more preferably from 1 to 10 and further more preferably from 2 to 5, in view of the stability of the oil-in-water emulsion, the stability of the slurry, the uniform appearance obtained, and the suppression of the separation and the reduction in viscosity after storage.

The content of the component (C) is 12 mass% or higher and 30 mass% or lower with respect to the total amount of the oil agent, in view of the stability of the oil-in-water emulsion, the stability of the slurry, and the provision of the solid powder cosmetic which is high in the surface uniformity of molded products, appropriate in terms of the amount taken upon application, very smooth and comfortable and gives no feeling of powder. Then, in view of the stability of the oil-in-water emulsion and the stability of the slurry, the content is preferably 12.5 mass% or higher, more preferably 13 mass% or higher, even more preferably 13.5 mass% or higher and even more preferably 14 mass% or higher; and in view of providing the solid powder cosmetic which is high in the surface uniformity of molded products, appropriate in terms of the amount taken upon application, very smooth and comfortable and gives no feeling of powder, the content is preferably 25 mass% or lower and more preferably 20 mass% or lower.

The content of the component (C) is preferably 0.9 mass% or higher and 3.9 mass% or lower with respect to the whole of powder, in view of the stability of the oil-in-water emulsion, the stability of the slurry, and the provision of the solid powder cosmetic which is high in the surface uniformity of molded products, appropriate in terms of the amount taken upon application, very smooth and comfortable and gives no feeling of powder. Then, in view of the stability of the oil-in-water emulsion and the stability of the slurry, the content is more preferably 1.4 mass% or higher, further more preferably 2.0 mass% or higher and even more preferably 2.5 mass% or higher; and in view of providing the solid powder cosmetic which is high in the surface uniformity of molded products, appropriate in terms of the amount taken upon application, very smooth and comfortable and gives no feeling of powder, the content is preferably 4.5 mass% or lower, more preferably 4.0 mass% or lower and further more preferably 3.5 mass% or lower.

The content of the component (C) is preferably 0.8 mass% or higher and 3.5 mass% or lower with respect to the total amount of the oil agent and the whole of powder, in view of the stability of the oil-in-water emulsion, the stability of the slurry, and the provision of the solid powder cosmetic which is high in the surface uniformity of molded products, appropriate in terms of the amount taken upon application, very smooth and comfortable and gives no feeling of powder. Then, in view of providing the solid powder cosmetic which is high in the surface uniformity of molded products, appropriate in terms of the amount taken upon application, very smooth and comfortable and gives no feeling of powder, the content is more preferably 1.0 mass% or higher, further more preferably 1.3 mass% or higher, even more preferably 1.7 mass% or higher and even more preferably 2.0 mass% or higher, and more preferably 3.2 mass% or lower and further more preferably 3.0 mass% or lower.

The solid powder cosmetic produced according to the present invention may also contain polyhydric alcohols, water-soluble polymers, beauty ingredients, perfumes, preservatives, ultraviolet absorbents, antioxidants and others, in addition to the powder, the oil agent and the surfactant.

The production of the solid powder cosmetic according to the present invention is characterized by mixing a powder containing the component (A) and the oil-in-water emulsion containing the components (B) and (C) and water as a solvent to make a slurry; packing the slurry in a cosmetic container; and thereafter, removing the solvent by suction. The method further comprises drying after removing the solvent by suction, which comprises drying at 20 to 95°C. In conventional production of a solid powder cosmetic, a method is adopted in which a powder containing the component (A) and an oil agent as the component (B) are first mixed and homogenized, and thereafter the mixture is dispersed in a solvent. By adopting the method of the present invention, there can be made a solid powder cosmetic which is high in the surface uniformity of molded products, appropriate in terms of the amount taken upon application, very smooth and excellent in the use impression.

First, a step of obtaining an oil-in-water emulsion containing the components (B) and (C) and water will be described.

In view of the handleability in the preparation and the stability of the emulsion, the amount of water to be used in preparation of an oil-in-water emulsion is preferably 50 mass% or higher, more preferably 60 mass% or higher and further more preferably 65 mass% or higher, and preferably 90 mass% or lower, more preferably 85 mass% or lower, further more preferably 82 mass% or lower, even more preferably 78 mass% or lower and even more preferably 75 mass% or lower, with respect to the whole of the emulsion. Here, the emulsion in the present invention contains water as a solvent, but may contain a water-soluble organic solvent as long as it does not inhibit emulsification. In the case of containing the water-soluble organic solvent, the content thereof is preferably 10 mass% or lower, more preferably 5 mass% or lower and further more preferably 0 mass%, with respect to water, in view of not inhibiting emulsification.

An apparatus for producing the oil-in-water emulsion is not especially limited as long as it is capable of producing a homogeneous oil-in-water emulsion, and it is preferable to use a homo mixer or an emulsifying apparatus having a disper blade. The circumferential speed of the stirring blade tip of the mixer in emulsification is preferably 1 m/s or higher, more preferably 3 m/s or higher and further more preferably 5 m/s or higher, and preferably 30 m/s or lower, more preferably 25 m/s or lower and further more preferably 20 m/s or lower, in view of good emulsification dispersibility and reduction in the load in the production.

In view of good emulsification dispersibility and reduction in the load in the production, the stirring time is preferably 1 min or longer, more preferably 3 min or longer and further more preferably 5 min or longer, and preferably 90 min or shorter, more preferably 75 min or shorter and further more preferably 60 min or shorter.

In view of good emulsification dispersibility and reduction in the load in the production, the stirring temperature is preferably 15°C or higher, more preferably 20°C or higher and further more preferably 25°C or higher, and preferably 90°C or lower, more preferably 80°C or lower and further more preferably 70°C or lower.

Then, the powder containing the component (A) and the oil-in-water emulsion are mixed to made a slurry.

An apparatus to be used in this step may be any apparatus as long as it is a mixing apparatus capable of homogeneously mixing the emulsion and the powder, and a high-speed dispersing machine, such as a homo disper having a disper blade, can be used.

In view of good emulsification dispersibility and reduction in the load in the production, the circumferential speed of the stirring blade tip of the mixing apparatus in the mixing preferably 1 m/s or higher, more preferably 3 m/s or higher and further more preferably 5 m/s or higher, and preferably 30 m/s or lower, more preferably 25 m/s or lower and further more preferably 20 m/s or lower.

In view of good emulsification dispersibility and reduction in the load in the production, the stirring time is preferably 1 min or longer, more preferably 3 min or longer and further more preferably 5 min or longer, and preferably 120 min or shorter, more preferably 75 min or shorter and further more preferably 60 min or shorter.

In view of good emulsification dispersibility and reduction in the load in the production, the stirring temperature is preferably 15°C or higher, more preferably 20°C or higher and further more preferably 25°C or higher, and preferably 90°C or lower, more preferably 80°C or lower and further more preferably 70°C or lower.

The obtained slurry is packed in a cosmetic container, and thereafter, the solvent is removed by suction. In the case of using an organic solvent as the solvent, the solvent is removed by suction in view of the safety and also in view of preventing the oil agent from otherwise being localized together with the solvent on the surface of the molded products by the capillary phenomenon in the case of only carrying out drying treatment. In the case of using water as the solvent, such problems do not arise. Further, it is technical common knowledge for those skilled in the art that when the solvent is removed by suction from an emulsion, the oil agent is also liable to be removed.

By contrast, in the present invention, it was found that in the case of using water as the solvent, the hardness of a cosmetic is increased and the disintegration resistance also is increased, by pressing the slurry while removing the solvent by suction. Thus, the present invention is characterized by carrying out removal by suction.

A cosmetic container to be used is a container suitable for the intended solid powder cosmetic.

A packing method is not especially limited, and either of front packing or back packing can be used.

With regard to the timing of removing the solvent by suction, the solvent may be removed by suction after the completion of packing of the slurry in a cosmetic container, or the slurry is packed in a cosmetic container while the solvent is removed by suction. Here, the degree of vacuum in removing the solvent by suction is preferably -5 kPa or higher and 80 kPa or lower, in view of the efficiency of the removal of the solvent and the load of the apparatus in the production. With regard to the content of the solvent after the solvent removal, it is preferable to carry out the removing until the content reaches 10% or higher and 25% or lower, in view of the efficiency of the removal of the solvent and the load of the apparatus in the production, and the drop-resistant strength of products. The content of the solvent is preferably 12% or higher, more preferably 15% or higher, further more preferably 19% or higher and even more preferably 20% or higher, and preferably 24% or lower and more preferably 23% or lower, in view of the efficiency of the removal of the solvent and the load of the apparatus in the production. Here, the solvent content is measured by a method described in Examples.

Further, the solid powder cosmetic is dried. Drying means is not especially limited, and may be natural drying, and hot air drying and vacuum drying can preferably be used.

In view of the drying efficiency, the load of the apparatus in the production and the thermal degradation of products, the drying temperature is 20°C or higher, preferably 25°C or higher and more preferably 30°C or higher, and 95°C or lower, preferably 90°C or lower and more preferably 85°C or lower.

The amount of moisture (moisture content) in the solid powder cosmetic after drying is preferably from 0.01 to 1.0 mass%, more preferably from 0.05 to 0.8 mass% and further more preferably from 0.1 to 0.5 mass%, in view of the amount taken upon application and the drop-resistant strength.

Examples of the solid powder cosmetic obtained by the method of the present invention includes makeup cosmetics such as eye colors, foundations, cheek blushes, eye liners, face powders and lip sticks.

With regard to the above-mentioned embodiments, the present invention further discloses the following production methods.

A method for producing a solid powder cosmetic comprising the following components (A) to (C):

(A) a color pigment in an amount of 0.1 mass% or higher and 34 mass% or lower in the whole of powder;
(B) an oil agent in an amount of 5 mass% or higher and 27 mass% or lower with respect to the whole of powder; and
(C) a surfactant in an amount of 12.5 mass% or higher and 25 mass% or lower with respect to the total amount of the oil agent,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

A method for producing a solid powder cosmetic comprising the following components (A) to (C):

(A) a color pigment in an amount of 0.1 mass% or higher and 34 mass% or lower in the whole of a powder;
(B) an oil agent in an amount of 5 mass% or higher and 27 mass% or lower with respect to the total amount of the powder; and
(C) a surfactant in an amount of 12 mass% or higher and 30 mass% or lower with respect to the total amount of the oil agent, and 0.8 mass% or higher and 3.5 mass% or lower with respect to the total amount of the oil agent and the whole of powder,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

A method for producing a solid powder cosmetic comprising the following components (A) to (C):

(A) a color pigment in an amount of 0.1 mass% or higher and 34 mass% or lower in the whole of powder;
(B) an oil agent in an amount of 5 mass% or higher and 27 mass% or lower with respect to the whole of powder; and
(C) a surfactant in an amount of 12 mass% or higher and 30 mass% or lower with respect to the total amount of the oil agent, and 0.9 mass% or higher and 3.9 mass% or lower with respect to the whole of powder,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

A method for producing a solid powder cosmetic comprising the following components (A) to (C):

(A) a color pigment in an amount of 0.1 mass% or higher and 34 mass% or lower in the whole of powder;
(B) an oil agent in an amount of 5 mass% or higher and 27 mass% or lower with respect to the whole of powder; and
(C) a surfactant in an amount of 12 mass% or higher and 30 mass% or lower with respect to the total amount of the oil agent, 0.8 mass% or higher and 3.5 mass% or lower with respect to the total amount of the oil agent and the whole of powder and 0.9 mass% or higher and 3.9 mass% or lower with respect to the whole of powder,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

A method for producing a solid powder cosmetic comprising the following components (A) to (C):

(A) a color pigment in an amount of 0.1 mass% or higher and 34 mass% or lower in the whole of powder;
(B) an oil agent in an amount of 3 mass% or higher and 28 mass% or lower with respect to the whole of powder; and
(C) a surfactant in an amount of 12.5 mass% or higher and 25 mass% or lower with respect to the total amount of the oil agent, 1.0 mass% or higher and 3.0 mass% or lower with respect to the total amount of the oil agent and the whole of powder and 1.4 mass% or higher and 3.5 mass% or lower with respect to the whole of powder,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

A method for producing a solid powder cosmetic comprising the following components (A) to (C):

(A) a color pigment in an amount of 0.1 mass% or higher and 34 mass% or lower in the whole of powder;
(B) an oil agent in an amount of 5 mass% or higher and 27 mass% or lower with respect to the whole of powder; and
(C) a surfactant in an amount of 12.5 mass% or higher and 25 mass% or lower with respect to the total amount of the oil agent, 1.0 mass% or higher and 3.0 mass% or lower with respect to the total amount of the oil agent and the whole of powder, and 1.4 mass% or higher and 3.5 mass% or lower with respect to the whole of powder,

the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

A method for producing a solid powder cosmetic comprising the following components (A) to (C):

(A) a color pigment in an amount of 0.1 mass% or higher and 34 mass% or lower in the whole of powder;
(B) an oil agent in an amount of 5 mass% or higher and 27 mass% or lower with respect to the whole of powder; and
(C) a surfactant in an amount of 12.5 mass% or higher and 25 mass% or lower with respect to the total amount of the oil agent,

the method comprising: mixing the powder comprising the component (A) and an oil-in-water emulsion comprising the components (B) and (C) and water to make a slurry; packing the slurry in a cosmetic container; thereafter, removing the solvent by suction so that the content of the solvent becomes 10 mass% or higher and 25 mass% or lower; and drying after removing the solvent by suction, which comprises drying at 20 to 95°C.

Examples

Then, the present invention will be described in more detail by way of Examples, but the present invention is not any more limited to these Examples.

[Methods for preparing molded products]

Examples 1 to 11 and Comparative Examples 1 to 5

Pure water was placed to a beaker and heated to 60°C, and a surfactant and an oil agent previously mixed were added thereto. Thereafter, the resultant was stirred by using a homo mixer MARKII 2.5 type (manufactured by Primix Corp.) at 60°C at 6,000 rpm for 5 min to thereby prepare an oil-in-water emulsion. A pigment was added to the emulsion, and the resultant was stirred by using a spatula until the pigment became moistened. Thereafter, the resultant was stirred by using a homo disper 2.5 type (manufactured by Primix Corp.) equipped with a disper blade with a size of 40 mm at 60°C at 4,200 rpm for 5 min to thereby obtain a slurry. 3 g of the obtained slurry was packed in a metal dish with a size of 27 mm × 27 mm, and twice pressed at a pressing pressure of 1 kN for 2 sec, with two sheets of cloth interposed between a pressing plate having a suction hole and the metal dish, while the solvent was removed by suction through the suction hole by vacuum. Thereafter, the resultant was dried at 40°C for 24 hours to thereby prepare a solid powder cosmetic; and the moisture content thereof after drying was measured.

Comparative Example 6

Pure water heated to 60°C, as well as a surfactant, an oil agent and a pigment were placed at once in a beaker, and stirred by a spatula until the pigment became moistened. Thereafter, the resultant was stirred by using a homo disper 2.5 type (manufactured by Primix Corp.) equipped with a disper blade with a size of 40 mm at 60°C at 4,200 rpm for 5 min to thereby obtain a slurry. 3 g of the obtained slurry was packed in a metal dish with a size of 27 mm × 27 mm, and twice pressed at a pressing pressure of 1 kN for 2 sec, with two sheets of cloth interposed between a pressing plate having a suction hole and the metal dish, while the solvent was removed by suction through the suction hole by vacuum. Thereafter, the resultant was dried at 40°C for 24 hours to thereby prepare a solid powder cosmetic; and the moisture content thereof after drying was measured.

[Method of measuring the hardness of the molded products (before drying)]

The hardness of the molded products was measured by using a RHEO METER COMPACT-100II (manufactured by Sun Scientific Co. Ltd.) under the condition of a penetration by a 2-mm$\phi$ jig of 1 mm.

[Method of measuring the moisture content of the molded products (before drying)]

The moisture content of the molded products (before drying) was calculated from the change in mass between the molded product (before drying) and the molded product (after drying). The drying condition was at 105°C for 3 hours.

Moisture content of molded product (before drying) = ((mass of molded product (before drying) - mass of molded product (after drying))/(mass of molded product (before drying))

[Method of evaluating the uniformity of the surface of the molded products]

The surface of the molded products (after drying) was observed by a microscope, and the height of roughness thereof was measured and evaluated based on the following point scores.
The uniformity of the surface of the molded products (after drying)

5 points: no roughness of 0.3 mm or more at maximum was found
4 points: roughness of 0.3 mm or more at maximum was found
3 points: roughness of 0.4 mm or more at maximum was found
2 points: roughness of 0.5 mm or more at maximum was found
1 point: roughness of 0.6 mm or more at maximum was found

[Method of sensory evaluation of the molded products (after drying)]

Five cosmetics researchers evaluated [amount taken upon application] and [comfortable feeling with less feeling of powder] of the prepared solid powder cosmetics, on the basis of a perfect score of 5 points; and their average point was taken as a point score of the solid powder cosmetic.

[Amount taken upon application]

5 points: the amount taken when the cosmetic was used with a chip was appropriate (Example 4).
4 points: the amount taken when the cosmetic was used with a chip was larger than that in Comparative Example 4.
3 points: the amount taken when the cosmetic was used with a chip was slightly larger than that in Comparative Example 4.
2 points: the amount taken when the cosmetic was used with a chip was quite slightly larger than that in Comparative Example 4.
1 point: the amount taken when the cosmetic was used with a chip was too small (Comparative Example 4).

[Comfortable feeling with less feeling of powder]

5 points: the cosmetic is very smooth and comfortable and almost no feeling of powder was perceived (Example 5).
4 points: as compared with Example 5, the cosmetic had slightly less smooth and comfortable feeling, and the feeling of powder was slightly perceived.
3 points: as compared with Example 5, the cosmetic had less smooth and comfortable feeling, and the feeling of powder was perceived.

2 points: as compared with Comparative Example 3, the feeling of powder was weak.

1 point: similarly, the feeling of powder was fairly strong (Comparative Example 3).

There are shown in Table 1, formulations, mass ratios of components blended, conditions for production and evaluation results of the molded products.

[Table 1]

| | | | Components, and the like | Ex 1 | Ex 2 | Ex 3 | Ex 4 | Ex 5 | Ex 6 | Ex 7 | Ex 8 | Ex 9 | Ex 10 | Ex 11 | CE 1 | CE 2 | CE 3 | CE 4 | CE 5 | CE 6 |
|---|---|---|---|---|---|---|---|---|---|---|---|---|---|---|---|---|---|---|---|---|
| Formulation [g] | Powder | Extender pigment | dimethicone-treated talc*1 | 17.50 | 17.50 | 20.00 | 22.50 | 22.50 | 25.00 | 17.50 | 17.50 | 17.50 | 17.50 | 17.50 | 17.50 | 17.50 | 17.50 | 17.50 | 22.50 | 22.50 |
| | | | methicone-treated sericite*2 | 17.50 | 17.50 | 20.00 | 22.50 | 22.50 | 25.00 | 17.50 | 17.50 | 17.50 | 17.50 | 17.50 | 17.50 | 17.50 | 17.50 | 17.50 | 22.50 | 22.50 |
| | | (A) Color pigment | dimethicone·2Na stearoylglutamate·Al hydroxide-treated red iron oxide*3 | 10.67 | 10.00 | 8.33 | 5.00 | 5.00 | 1.67 | 10.67 | 10.67 | 10.67 | 10.67 | 10.67 | 10.67 | 11.67 | 10.67 | 10.67 | 5.00 | 5.00 |
| | | | dimethicone·2Na stearoylglutamate·Al hydroxide-treated yellow iron oxide*4 | 10.67 | 10.00 | 8.33 | 5.00 | 5.00 | 1.67 | 10.67 | 10.67 | 10.67 | 10.67 | 10.67 | 10.67 | 11.67 | 10.67 | 10.67 | 5.00 | 5.00 |
| | | | dimethicone·2Na stearoylglutamate·Al hydroxide-treated black iron oxide*5 | 10.67 | 10.00 | 8.33 | 5.00 | 5.00 | 1.67 | 10.67 | 10.67 | 10.67 | 10.67 | 10.67 | 10.67 | 11.67 | 10.67 | 10.67 | 5.00 | 5.00 |
| | | Glittering pigment | titanium oxide-coated synthetic mica*6 | 33.00 | 35.00 | 35.00 | 40.00 | 40.00 | 45.00 | 33.00 | 33.00 | 33.00 | 33.00 | 33.00 | 33.00 | 30.00 | 33.00 | 33.00 | 40.00 | 40.00 |
| | | | Total | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| | Emulsion | | (C) polyoxyethylene sorbitol tetraoleate*7 | 3.51 | 3.51 | 3.51 | 3.51 | 3.70 | 3.51 | 2.85 | 2.19 | 1.76 | 1.32 | 0.66 | 1.10 | 3.51 | 3.95 | 0.66 | 3.51 | 3.51 |
| | | | (B) hydrogenated polydecene*8 | 16.11 | 16.11 | 16.11 | 16.11 | 16.11 | 16.11 | 13.09 | 10.07 | 8.05 | 6.04 | 3.02 | 6.04 | 16.11 | 18.12 | 3.02 | 16.11 | 16.11 |
| | | | (B) α-olefin oligomer*9 | 6.43 | 6.43 | 6.43 | 6.43 | 6.43 | 6.43 | 5.22 | 4.02 | 3.21 | 2.41 | 1.21 | 2.41 | 6.43 | 7.23 | 1.21 | 6.43 | 6.43 |
| | | | (B) hydrogenated polyisobutene*10 | 3.21 | 3.21 | 3.21 | 3.21 | 3.21 | 3.21 | 2.61 | 2.01 | 1.61 | 1.21 | 0.60 | 1.21 | 3.21 | 3.62 | 0.60 | 3.21 | 3.21 |
| | | | distilled water | 64.63 | 64.63 | 64.63 | 64.63 | 64.40 | 64.63 | 61.89 | 59.15 | 61.83 | 55.49 | 52.74 | 55.69 | 62.74 | 66.46 | 52.74 | 64.63 | 64.63 |
| | | | Total | 93.89 | 93.89 | 93.89 | 93.89 | 93.85 | 93.89 | 85.67 | 77.44 | 76.46 | 66.46 | 58.23 | 66.44 | 92.00 | 99.38 | 58.23 | 93.89 | 93.89 |
| Blend mass ratio [mass%] | | | Emulsion liquid/Powder | 93.9 | 93.9 | 93.9 | 93.9 | 93.8 | 93.9 | 85.7 | 77.4 | 76.5 | 66.5 | 58.2 | 66.4 | 92.0 | 99.4 | 58.2 | 93.9 | 93.9 |
| | | | Oil agent/Powder | 25.7 | 25.7 | 25.7 | 25.7 | 25.7 | 25.7 | 20.9 | 16.1 | 12.9 | 9.7 | 4.8 | 9.7 | 25.7 | 29.0 | 4.8 | 25.7 | 25.7 |
| | | | Color pigment/Powder | 32.0 | 32.0 | 25.0 | 15.0 | 15.0 | 5.0 | 32.0 | 32.0 | 32.0 | 32.0 | 32.0 | 32.0 | 35.0 | 32.0 | 32.0 | 15.0 | 15.0 |
| | | | [Surfactant + Oil agent]/Powder | 29.3 | 29.3 | 29.3 | 29.3 | 29.4 | 29.3 | 23.8 | 18.3 | 14.6 | 11.0 | 5.5 | 10.8 | 29.3 | 32.9 | 5.5 | 29.3 | 29.3 |
| | | | Surfactant/Oil agent | 13.6 | 13.6 | 13.6 | 13.6 | 14.4 | 13.6 | 13.6 | 13.6 | 13.6 | 13.6 | 13.6 | 11.4 | 13.6 | 13.6 | 13.6 | 13.6 | 13.6 |
| | | | Surfactant/[Powder + Oil agent] | 2.8 | 2.8 | 2.8 | 2.8 | 2.9 | 2.8 | 2.4 | 1.9 | 1.6 | 1.2 | 0.63 | 1.0 | 2.8 | 3.1 | 0.6 | 2.8 | 2.8 |
| | | | Surfactant/Powder | 3.5 | 3.5 | 3.5 | 3.5 | 3.7 | 3.5 | 2.9 | 2.2 | 1.8 | 1.3 | 0.66 | 1.1 | 3.5 | 4.0 | 0.7 | 3.5 | 3.5 |
| Conditions for production | | | Emulsification | Yes | Yes | Yes | Yes | Yes | Yes | Yes | Yes | Yes | Yes | Yes | Yes | Yes | Yes | Yes | Yes | No |
| | | | Suction molding | Yes | Yes | Yes | Yes | Yes | Yes | Yes | Yes | Yes | Yes | Yes | Yes | Yes | Yes | No | No | Yes |
| | | | Conditions for drying | 40°C 24h | 40°C 24h | 40°C 24h | 40°C 24h | 40°C 24h | 40°C 24h | 40°C 24h | 40°C 24h | 40°C 24h | 40°C 24h | 40°C 24h | 40°C 24h | 40°C 24h | 40°C 24h | 40°C 24h | 40°C 24h | 40°C 24h |
| Component composition in cosmetic after production [mass%] | | | Powder | 77.2 | 77.1 | 77.1 | 77.1 | 77.0 | 77.2 | 80.6 | 84.3 | 87.0 | 89.9 | 94.6 | 90.0 | 77.2 | 75.0 | 94.5 | 77.2 | 77.1 |
| | | | Moisture content | 0.24 | 0.31 | 0.29 | 0.28 | 0.30 | 0.27 | 0.28 | 0.27 | 0.25 | 0.29 | 0.26 | 0.28 | 0.27 | 0.29 | 0.28 | 0.27 | 0.28 |
| Evaluations | | | Water content of molded product (before dried) [mass%] | 22.3 | 21.4 | 21.8 | 18.3 | 18.9 | 15.4 | 20.4 | 19.5 | 19.3 | 18.8 | 18.4 | 19.0 | 28.4 | 27.8 | 33.3 | 33.3 | 19.0 |
| | | | Hardness of molded product (before dried) [gf] | 53 | 68 | 81 | 215 | 199 | 320 | 91 | 180 | 470 | 500 | 520 | 489 | 0 | 0 | - | - | 230 |
| | | | Uniformity of surface of molded product | 5.0 | 5.0 | 5.0 | 5.0 | 5.0 | 5.0 | 5.0 | 5.0 | 5.0 | 5.0 | 5.0 | 2.0 | 2.0 | 2.0 | 1.0 | 1.0 | 2.0 |
| | | | Amount taken upon application | 4.0 | 4.2 | 4.6 | 5.0 | 5.0 | 5.0 | 4.0 | 3.8 | 3.8 | 3.6 | 3.4 | 2.4 | 2.2 | 2.0 | 1.4 | 1.0 | 2.0 |
| | | | Comfortable feeling with less feeling of powder | 3.8 | 4.2 | 4.4 | 4.6 | 4.8 | 5.0 | 3.8 | 3.6 | 3.4 | 3.2 | 3.0 | 2.0 | 1.6 | 1.6 | 1.0 | 1.0 | 1.0 |

*1: talc JA-68R (Asada Milling Co. Ltd.)

*2: sericite FSE (Sanshin Koko KK)

*3: synthetic iron oxide R-516P (Titan Kogyo, Ltd.)

*4: synthetic iron oxide LL-100 (Titan Kogyo, Ltd.)

*5: synthetic iron oxide BL-100P (Titan Kogyo, Ltd.)

*6: Metashine MT1040RS (Nippon Sheet Glass Co. Ltd.)

*7: Rheodol 430V LOT2085 (Kao Corp.)

*8: Silkflo 364 (Vantage Specialty Chemicals, Inc.)

*9: Nomcort HP-100 (Nisshin Oillio Group, Ltd.)

*10: P-RC-10SH (NOF Corp.)

From Table 1, it is clear that the solid powder cosmetic produced by mixing a powder containing the component (A) with an oil-in-water emulsion containing the component (B) (3 mass% or higher and 28 mass% or lower with respect to the whole of powder), the component (C) (12 mass% or higher and 30 mass% or lower with respect to the total amount of the oil agent) and water to make a slurry, packing the slurry in a cosmetic container and thereafter removing the solvent by suction was a solid powder cosmetic which was high in the uniformity of the surface of the molded product, appropriate in terms of the amount taken upon application, very smooth and comfortable and gave no feeling of powder, as compared with the cosmetic too much in the oil agent, the cosmetic little in the surfactant and the cosmetic obtained by not removing solvent by suction.

**Claims**

1. A method for producing a solid powder cosmetic comprising the following components (A) to (C):

   (A) a color pigment in an amount of 0.1 mass% or higher and 34 mass% or lower in the whole of powder;
   (B) an oil agent in an amount of 3 mass% or higher and 28 mass% or lower with respect to the whole of powder; and
   (C) a surfactant in an amount of 12 mass% or higher and 30 mass% or lower with respect to the total amount of the oil agent,

   the method comprising: mixing the powder comprising the component (A) and an oil-in-water emulsion comprising the components (B) and (C) and water to make a slurry; packing the slurry in a cosmetic container; thereafter, removing the solvent by suction; and drying after removing the solvent by suction, which comprises drying at 20 to 95°C.

2. The method for producing a solid powder cosmetic according to claim 1, wherein a content of the component (C) is 0.8 mass% or higher and 3.0 mass% or lower with respect to the total amount of the oil agent and the whole of powder.

3. The method for producing a solid powder cosmetic according to claim 1 or 2, wherein a content of the component (C) is 0.9 mass% or higher and 3.9 mass% or lower with respect to the whole of powder.

4. The method for producing a solid powder cosmetic according to any one of claims 1 to 3, wherein the removing the solvent by suction is carried out so that a content of the solvent becomes 10 mass% or higher and 25 mass% or lower.

5. The method for producing a solid powder cosmetic according to any one of claims 1 to 4, wherein a circumferential speed of a stirring blade tip is 1 m/s or higher and 30 m/s or lower when the powder comprising the component (A) and the oil-in-water emulsion comprising the components (B) and (C) and water are mixed.

**Patentansprüche**

1. Verfahren zur Herstellung eines festen Kosmetikums mit Pulver, umfassend die folgenden Komponenten (A) bis (C):

   (A) ein Farbpigment in einer Menge von 0,1 Massen-% oder mehr und 34 Massen-% oder weniger in der Gesamtheit des Pulvers;
   (B) ein Ölmittel in einer Menge von 3 Massen-% oder mehr und 28 Massen-% oder weniger, bezogen auf die Gesamtheit des Pulvers; und
   (C) ein Tensid in einer Menge von 12 Massen-% oder mehr und 30 Massen-% oder weniger, bezogen auf die Gesamtmenge des Ölmittels,

   wobei das Verfahren umfasst: das Mischen des Pulvers, umfassend die Komponente (A), und einer Öl-in-Wasser-Emulsion, umfassend die Komponenten (B) und (C) und Wasser, um eine Aufschlämmung herzustellen; das Packen der Aufschlämmung in einen Kosmetikbehälter; das anschließende Entfernen des Lösungsmittels durch Saugen; und das Trocknen nach dem Entfernen des Lösungsmittels durch Saugen, das ein Trocknen bei 20 bis 95°C umfasst.

2. Verfahren zur Herstellung eines festen Kosmetikums mit Pulver gemäß Anspruch 1, wobei ein Gehalt der Komponente (C) 0,8 Massen-% oder mehr und 3,0 Massen-% oder weniger, bezogen auf die Gesamtmenge des Ölmittels und der Gesamtheit des Pulvers, beträgt.

3. Verfahren zur Herstellung eines festen Kosmetikums mit Pulver gemäß Anspruch 1 oder 2, wobei ein Gehalt der Komponente (C) 0,9 Massen-% oder mehr und 3,9 Massen-% oder weniger, bezogen auf die Gesamtheit des Pulvers, beträgt.

4. Verfahren zur Herstellung eines festen Kosmetikums mit Pulver gemäß einem der Ansprüche 1 bis **3,** wobei das Entfernen des Lösungsmittels mittels Saugen so durchgeführt wird, dass ein Gehalt des Lösungsmittels 10 Massen-% oder mehr und 25 Massen-% oder weniger beträgt.

5. Verfahren zur Herstellung eines festen Kosmetikums mit Pulver gemäß einem der Ansprüche 1 bis **4,** wobei eine Umfangsgeschwindigkeit einer Rührflügelspitze 1 m/s oder mehr und 30 m/s oder weniger beträgt, wenn das Pulver, umfassend die Komponente (A), und die Öl-in-Wasser-Emulsion, umfassend die Komponenten (B) und (C) und Wasser, gemischt werden.

**Revendications**

1. Procédé de production d'un produit cosmétique en poudre solide comprenant les composants (A) à (C) suivants :

   (A) un pigment de couleur dans une quantité de 0,1 % en masse ou plus et 34 % en masse ou moins dans l'ensemble de la poudre ;
   (B) un agent huileux dans une quantité de 3 % en masse ou plus et 28 % en masse ou moins par rapport à l'ensemble de la poudre ; et
   (C) un tensioactif dans une quantité de 12 % en masse ou plus et 30 % en masse ou moins par rapport à la quantité totale de l'agent huileux,

   le procédé comprenant : le mélange de la poudre comprenant le composant (A) et d'une émulsion huile dans eau comprenant les composants (B) et (C) et de l'eau pour fabriquer une suspension ; l'emballage de la suspension dans un récipient pour produit cosmétique ; par la suite, l'élimination du solvant par aspiration ; et le séchage après élimination du solvant par aspiration, qui comprend un séchage à 20 °C à 95 °C.

2. Procédé de production d'un produit cosmétique en poudre solide selon la revendication 1, dans lequel une teneur du composant (C) est 0,8 % en masse ou plus et 3,0 % en masse ou moins par rapport à la quantité totale de l'agent huileux et de l'ensemble de la poudre.

3. Procédé de production d'un produit cosmétique en poudre solide selon la revendication 1 ou la revendication 2, dans lequel une teneur du composant (C) est 0,9 % en masse ou plus et 3,9 % en masse ou moins par rapport à l'ensemble de la poudre.

4. Procédé de production d'un produit cosmétique en poudre solide selon l'une quelconque des revendications 1 à 3, dans lequel l'élimination du solvant par aspiration est réalisée de sorte qu'une teneur du solvant devienne 10 % en masse ou plus et 25 % en masse ou moins.

5. Procédé de production d'un produit cosmétique en poudre solide selon l'une quelconque des revendications 1 à 4, dans lequel une vitesse circonférentielle d'un bout de pale d'agitation est 1 m/s ou plus et 30 m/s ou moins lorsque la poudre comprenant le composant (A) et l'émulsion huile dans eau comprenant les composants (B) et (C) et de l'eau sont mélangées.

**EP 4 279 055 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5958389 A
- US 2018028414 A1
- JP 2020183352 A
- JP 2010047528 A
- JP 2009242282 A